(19) Europäisches Patentamt
European Patent Office
Office européen des brevets

(11) Veröffentlichungsnummer: **0 275 961 B1**

(12) # EUROPÄISCHE PATENTSCHRIFT

(45) Veröffentlichungstag der Patentschrift: **08.06.94**

(51) Int. Cl.[5]: **A61K 9/22**, A61K 9/52

(21) Anmeldenummer: **88100636.5**

(22) Anmeldetag: **19.01.88**

(54) **Perorale Arzneimittelzubereitung mit verzögerter Wirkstofffreigabe.**

(30) Priorität: **21.01.87 DE 3701625**

(43) Veröffentlichungstag der Anmeldung:
**27.07.88 Patentblatt 88/30**

(45) Bekanntmachung des Hinweises auf die Patenterteilung:
**08.06.94 Patentblatt 94/23**

(84) Benannte Vertragsstaaten:
**AT BE CH DE ES FR GB GR IT LI LU NL SE**

(56) Entgegenhaltungen:
**EP-A- 0 058 481**
**EP-A- 0 147 335**
**DE-A- 2 824 112**
**FR-A- 2 070 153**
**FR-A- 2 390 962**

(73) Patentinhaber: **BOEHRINGER INGELHEIM KG**

**D-55216 Ingelheim(DE)**
(84) Benannte Vertragsstaaten:
**BE CH DE ES FR GR IT LI LU NL SE AT**

(73) Patentinhaber: **BOEHRINGER INGELHEIM INTERNATIONAL GmbH**

**D-55216 Ingelheim(DE)**

(84) Benannte Vertragsstaaten:
**GB**

(72) Erfinder: **Zierenberg, Bernd, Dr.**
**Goethestrasse 1**
**D-6530 Bingen(DE)**
Erfinder: **Freund, Bernhard, Dr.**
**Karl-Domdey-Strasse 28**
**D-6535 Gau-Algesheim(DE)**
Erfinder: **Bendix, Dieter, Dr.**
**Veit-Stoss-Strasse 17**
**D-6507 Ingelheim am Rhein(DE)**
Erfinder: **Entenmann, Günther, Dr.**
**Veit-Stoss-Strasse 17**
**D-6507 Ingelheim am Rhein(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 275 961 B1

## Beschreibung

Die Erfindung betrifft perorale Arzneimitteldarreichungsformen mit verzögerter Wirkstoffreisetzung.

Perorale Arzneimittelformen, auch mit verzögerter Wirkstofffreigabe, auf der Basis von polymeren Wirkstoffträgern sind in einer Vielzahl von Patentschriften beschrieben. Als polymere Trägerstoffe gelangen beispielsweise Polymere auf der Basis von Acrylsäuren, Cellulosen u.a. zum Einsatz. Als nachteilig wird hierbei empfunden, daß es sich bei den meisten Trägermaterialien um nichtphysiologische Substanzen handelt, die in eingehenden Studien auf ihre toxikologische Unbedenklichkeit untersucht werden müssen.

Oligomere, Polymere und Copolymere von niederen Hydroxycarbonsäuren, insbesondere von Glycolsäure und Milchsäure, werden im Körper duch den natürlichen Stoffwechselkreislauf abgebaut und sind aus toxikologischer Sicht unbedenklich. Naht- und Implantationsmaterialien auf Basis dieser Polymere werden bereits seit längerem erfolgreich in der Chirurgie eingesetzt.

Parenterale Arzneimittelzubereitungen aus Copolymeren der Milch- und Glycolsäure werden beispielsweise in der Europäischen Patentanmeldung 26 599 wie auch in der DE-OS 20 51 580 offenbart. Es werden dort Arzneimittelzubereitungen aus diesen Polymeren beschrieben, die die Eigenschaft besitzen, den Wirkstoff über einen längeren Zeitraum, d.h. zwischen 14 Tagen und einem Jahr abzugeben. Dies ist bei parenteralen Depotformen erwünscht, läßt jedoch aufgrund der langen Freisetzungsrate Arzneimittelzubereitungen auf Basis von Polymeren der Milch- und Glycolsäure für orale Arzneimittelzubereitungen als völlig ungeeignet erscheinen.

Aus der europäischen Patentanmeldung 0 147 335 ist ferner eine inerte pharmazeutische Matrix auf der Basis von Caprolacton bekannt. Diese Matrix basiert auf einem inerten Material, d.h. einem Material, das im menschlichen oder tierischen Organismus nicht aufgelöst wird und die in keiner Weise mit den Wirkstoffen oder den im Körper vorhandenen Flüssigkeiten in Interaktion treten kann. Die Abgabe des Wirkstoffes erfolgt bei diesem Matrixsystem aus den Hohlräumen des porösen Matrixmaterials.

Die deutsche Offenlegungsschrift 2 824 112 betrifft Mikropellets und Verfahren zu ihrer Herstellung. Dabei wird beispielsweise ein pharmazeutischer Wirkstoff von einem bioabbaubaren Polymer eingehüllt, wobei dieses Polymer oder Copolymer u.a. aus Polymilchsäure oder Polyglycolsäure aufgebaut sein kann. Das in dieser Offenlegungsschrift offenbarte Verfahren ist jedoch relativ kompliziert.

Die französische Offenlegungsschrift 23 90 962 betrifft daneben ein Arzneimittel mit Depotwirkung für die parenterale Gabe von Arzneistoffen.

Die Anforderungen, die an parenteral zu verabreichende Arzneimittelzubereitungen gestellt werden unterscheiden sich jedoch grundlegend von denjenigen Anforderungen, die aus peroral zu verabreichende Wirkstofffreigabesysteme gestellt werden.

Es ist die Aufgabe der vorliegenden Erfindung eine perorale Applikationsform mit verzögerter Wirkstofffreisetzung vorzuschlagen, die aus einem toxisch unbedenklichem Polymermaterial besteht und gleichzeitig eine breite Variation der Freisetzungsrate unter Verwendung der gleichen "Basispolymere" ermöglicht.

Erfindungsgemäß wird eine wirkstoffhaltige perorale Applikationsform in Form einer Tablette, eines Pulvers, eines Granulates, einer Kapsel oder einer Pelletform auf Basis von biologisch abbaubaren cyclischen Carbonsäureestern, deren Oligomere, Co-Oligomere, deren Homo- und/oder Copolymeren mit einer kontinuierlichen Wirkstofffreigabe innerhalb von 24 Stunden vorgeschlagen.

Geeignete Trägermaterialien oder Überzugsmaterialien hierfür sind Glykolid und/oder Lactid, L-Lactid, D-Lactid, D,L-Lactid, meso-Lactid, Poly-L-Lactid, Poly-D-Lactid, Poly-D,L-Lactid, Poly(L-Lactid-co-glycolid) und Poly(D,L-Lactid-co-glycolid) oder einer oder mehrerer Kombinationen daraus.

Geeignete Trägermaterialien bzw. Überzugsmaterialien sind die oben beschriebenen Lactone selbst, ihre Oligomeren und Co-Oligomeren mit Molekulargewichten bis 3000 sowie ihre Polymeren und Copolymeren mit Molekulargewichten bis zu 1,2 Mio.. Die Molekulargewichte beziehen sich auf das Zahlenmittel. Geeignete Trägermaterialien sind weiter Gemische der oben beschriebenen Lactone, ihrer Oligomerer und Co-Oligomerer, Polymerer und Co-polymerer untereinander.

Geeignete Trägermaterialien sind weiter Gemische der hier beschriebenen Lactone, Oligomeren und Polymeren mit anderen Polymeren wie Polyacrylaten, Polymethacrylaten, Polyestern, Polyamiden, Cellulosen und Stärken.

Einige der oben beschriebenen Lactone sowie die daraus hergestellten Oligomeren und Polymeren weisen Asymmetriezentren auf. Geeignete Ausgangsmaterialien zur Herstellung der Trägermaterialien sind die rechtsdrehenden Formen, die linksdrehenden Formen, die optisch inaktiven racemischen Formen, die optisch inaktiven meso-Formen sowie Gemische beliebiger Zusammensetzung aus den einzelnen Formen.

Co-Oligomere und Copolymere aus den beschriebenen Lactonen können wahlweise so hergestellt werden, daß die verschiedenen Monomereinheiten statistisch verteilt oder in Blöcken unterschiedlicher Länge in der Polymerkette auftreten. Geeignete Trägermaterialien sind sowohl statistische Co-Oligomere

und Copolymere als auch Block-co-oligomere und -polymere.

Die beschriebenen Oligomere, Polymere und Copolymeren können auf verschiedene Arten hergestellt werden. In verschiedenen Patentschriften ist die Kondensation von Hydroxycarbonsäuren, in anderen Patentschriften die ringöffnende Polymerisation von Lactonen beschrieben, zum Beispiel:

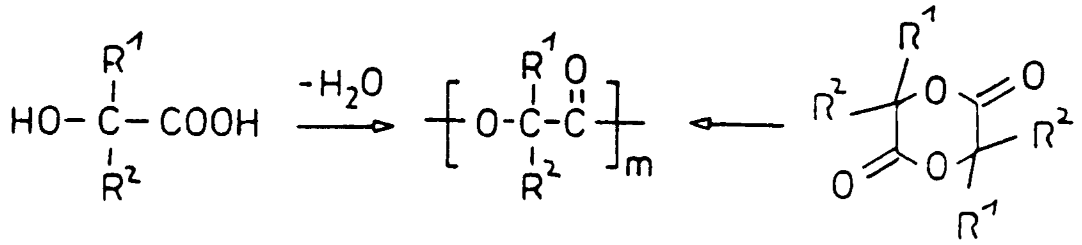

Für die Verwendung als Trägermaterial für Arzneimittelzubereitungen ist die Herstellung der genannten Oligomeren, Polymeren und Copolymeren im allgemeinen nicht kritisch. Geeignet und damit Gegenstand der Erfindung sind die Oligomeren, Co-Oligomeren, Polymeren und Copolymeren unabhängig davon, ob sie durch Kondensation aus Hydroxysäuren und ihren Derivaten oder durch Polymerisation aus Lactonen hergestellt werden. Der Einfachheit halber werden die Oligomeren, Co-Oligomeren, Polymeren und Copolymeren als Polymere der Lactone bezeichnet, ohne daß damit auf die Herstellungsweise, den Polymerisationsgrad oder andere Eigenschaften bezug genommen wird.

Bevorzugte Trägermaterialien bzw. Überzugsmaterialien sind Polymere und Copolymere aus Glycolid, L-Lactid, D,L-Lactid, D-Lactid, Die Copolymeren können aus zwei oder mehreren Comonomeren aufgebaut sein. Der Gehalt an jedem Comonomeren kann zwischen 1 und 99% variieren.

Besonders bevorzugt Lactone sind
Glycolid
L-Lactid
D-Lactid
D,L-Lactid (Racemat)
meso-Lactid
Besonders bevorzugte Polymere sind
Poly-L-Lactid
Poly-D-Lactid
Poly-D,L-Lactid
Poly (L-Lactid-co-glycolid) mit ≦ 55 Mol-%
Glycolid
Poly (D,L-Lactid-co-glycolid) mit ≦ 55 Mol-%
Glycolid

Zur Erreichung der gewünschten Freigaberate können die erfindungsgemäßen Arzneimittelzubereitungen beispielsweise nach den folgenden Verfahren hergestellt werden:

Ein wie zuvor definiertes Polymer oder Copolymer wird in einem leichtflüchtigen Lösungsmittel, wie z.B. Dichlormethan, gelöst bzw. suspendiert und mit dem Wirkstoff versetzt. (Bei diesen Herstellungsverfahren können selbstverständlich nur solche Polymere bzw. Copolymere verwendet werden, die sich in einem leichtverdampfbaren Lösungsmittel hinreichend auflösen oder zumindes aufquellen.) Die Löslichkeit eines Copolymers aus Glycolid und Lactid sinkt mit steigenden Glycolidanteil. Copolymere mit über 55 % Glycolidanteil lösen sich nicht mehr in den in Frage kommenden leichtflüchtigen Lösungsmitteln. Die bei diesem Verfahren erhaltene Lösung oder Suspension wird zu einem Film ausgegossen und das Lösungsmittel entfernt. Anschließend wird der dabei entstehende Film unterhalb der Glastemperatur des jeweiligen Polymeren auf eine Korngröße zwischen 10 und 500 µm vermahlt. Eine Auswahl der Korngröße ermöglicht eine zusätzliche Variation der Freisetzungsgeschwindigkeit. Das so erhaltene Pulver kann entweder direkt zu Tabletten verpreßt oder in Kapseln gefüllt werden. Das Verhältnis zwischen Polymeren und Wirkstoff ist in weiten Bereichen unkritisch und liegt zweckmäßigweise zwischen 0.1 und 20 %, bevorzugt 0.5 und 5 %. Wenn es erforderlich erscheint, können bei der Herstellung der Arzneimittelzubereitung, d.h. bei der Kaltvermahlung oder dem Tablettenpressen in der Pharmazie übliche Hilfstoffe, wie z.B. Maisstärke, Lactose u.a., beigemengt werden. Bei der Kaltvermahlung kann das Massenverhältnis zwischen wirkstoffhaltigem Polymerisat und Hilfstoff innerhalb weiter Grenzen schwanken und etwa bei 10 : 1 bis 1 : 4 liegen.

Ein bevorzugtes Polymer ist ein Poly-DL-Lactid mit einem Polymerisationsgrad zwischen 4 und 6. Weitere bevorzugte Polymere sind Poly-L-Lactid mit einem mitleren Molekulargewicht von ca. 2.000, Poly-L-Lactid (MG ca 43000), Poly-D,L-Lactid (MG ca. 36 3000), Copolymer D,L-Lactid/Glycolid 50/50 (MG ca. 8000) sowie ein Copolymer aus D,L-Lactid/Glycolid 75/25 mit einem mittleren Molekulargewicht von ca. 19 000.

Bei einer nach diesem Verfahren hergestellten oralen Arzneimittelzubereitung wird während der Verweildauer im Magen praktisch kein Arzneimittel freigesetzt, obwohl die Tablette im Magen sehr schnell in einzelne Partikel zerfällt. Die Freigabe erfolgt erst während der Darmpassage, d.h. im neutralen Milieu, wobei dann der Arzneistoff innerhalb von drei bis zu 12 Stunden kontinuierlich freigesetzt wird. Die beschriebenen Eigenschaften eröffnen für die erfindungsgemäße Arzneimittelzubereitung die Möglichkeit einer pH-abhängigen, d.h. mit steigendem pH-Wert zunehmenden Freisetzung von Wirkstoffen, wie z.B. eine gezielte Wirkstofffreigabe im Darmtrakt. Ein Anwendungsgebiet ist beispielsweise auch die magensaftresistente Depottablette.

Niedermolekulares Poly-D,L-Lactid weist die höchsten Freigaberaten auf. Die Freigabe kann durch Zumischung von höher molekularen Polymeren wie auch Copolymeren Lactiden bzw. Glycolide kontinuierlich verlängert werden, so daß eine individuelle Anpassung der erfindungsgemäßen Arzneimittelzubereitung an den Wirkstoff und an die Therapieerfordernisse möglich ist.

Im Gegensatz dazu bewirkt die Zumischung von anderen Polymeren, z.B. Polyacrylaten (z.B. Eudragit$^R$ NE 30 D) eine Erhöhung der Freisetzungsrate.

Ein weiterer Typ der erfindungsgemäßen Arzneimittelzubereitung sind Tabletten vom Matrixtyp. Zur Herstellung einer Matrixtablette wird der Wirkstoff zusammen mit dem Polymeren bzw. Copolymeren oder deren Gemisch, gegebenenfalls unter Verwendung geringer Mengen in der Pharmazie üblicher Hilfstoffe, wie z.B. Magnesiumstearat, Aerosil u.a., nach bekannten Verfahren, z.B. aus einem durch Feuchtgranulation gebildetem Granulat hergestellt oder aber auch durch Direktverpressung von Polymermaterial und Wirkstoff.

Nachfolgend sind einige Beispiele für Polymere und Copolymere genannt, welche sich zur Herstellung von Tabletten des Matrixtyps besonders eignen:

| | |
|---|---|
| Poly (L-lactid), | Molekulargewicht zwischen 1000 und 5000 (bestimmt durch Endgruppentitration |
| Poly (L-lactid), | inhärente Viskosität zwischen 0,5 und 1,5 |
| Poly (D,L-lactid) | inhärente Viskosität zwischen 1,5 und 2,5 |
| Poly (D,L-lactid-co-glycolid) | 50:50, inhärente Viskosität zwischen 0,2 und 1,0 |
| Poly (D,L-lactid-co-glycolid) | 75:25, inhärente Viskosität zwischen 0,2 und 1,0 |

Besonders bevorzugt sind die nachfolgend aufgeführte Polymere und Copolymere

Poly-L-Milchsäure M = 2000
Poly-L-lactid $M_{vis}$ = 43.000
Poly-D,L-lactid $M_{vis}$ = 363.000
Copolymere [Poly(D,L-Lactid-co-glycolid)] 50:50
Copolymere [Poly(D,L-Lactid-co-glycolid)] 75:25

Die dargestellte Auswahl trifft ebenfalls für andere in dieser Anmeldung beschriebenen Arzneimittelformen zu.

Diese Aufzählung ist beispielhaft zu verstehen, ohne die Erfindung dabei auf die genannten Polymeren oder Molekulargewichte oder inhärenten Viskositäten einzuschränken.

Die erfindungsgemäße Matrixtablette zeichnet sich durch einen sehr hohen Wirkstoffgehalt aus, der in Abhängigkeit vom Wirkstoff bis zu 90 % betragen kann. Als untere Grenze wird ein Gehalt von 40 % an Wirkstoff angesehen.

Bei sehr aktiven Arzneimittelwirkstoffen ist es manchmal notwendig, geringere als die oben angegebenen Wirkstoffmengen in einer Matrixtablette zu verarbeiten; in diesem Fall ist es sehr vorteilhaft zusätzlich ein leicht löslichen Hilfstoff, wie z.B. Lactose, oder auch in Wasser schlechter bzw. unlösliche Hilfsstoffe mitzuverarbeiten, um eine Wirkstofffreigabe innerhalb von 24 Stunden zu gewährleisten.

Im Gegensatz zu den zuvor beschriebenen schnell zerfallenden Depotabletten erfolgt bei der erfindungsgemäßen Matrixtablette die Wirkstofffreisetzung bereits im Magenbereich. Neben der Polymerzusammensetzung bestimmt auch die Form der Matrixtablette das Freigabeverhalten. Eine Erhöhung des Molekulargewichtes bewirkt eine Verringerung der Freisetzungsrate.

Eine weitere Ausführung der erfindungsgemäßen Arzneimittelzubereitung sind perorale Darreichungsformen mit einem retardierenden Überzug bestehend aus einem Homo- oder Copolymer analog - der zuvor beschriebenen Trägermaterialien der Strukturen I, II, III oder IV - bevorzugt aus einem Lactid/Glycolid Homo- oder Copolymeren, gegebenenfalls in Kombination mit anderen Polymeren, wie z.B. wasserlöslichen Polymeren, wie z.B. Polyethylenglykol oder wasserquellbaren Polymeren wie z.B. Eudragit$^R$ NE 30 D.

Die Technik zur Herstellung von retardierenden Überzügen ist dem Fachmann hinreichend bekannt und bedarf keiner besonderen Erläuterungen.
So wird beispielweise der Wirkstoff in Form von Pellets (ca. 1.0-1.6 mm) in einem Wirbelschichtgranulierer mit dem in einem geeigneten Lösungsmittel gelösten Polymer überzogen. Entsprechende Überzüge können auch nach dem Dragierkesselverfahren aufgebracht werden. Im allgemeinen beträgt die Menge des retardierenden Überzugs 3 bis 30 Gew.%, bevorzugt 5 bis 20 besonders bevorzugt 5 bis 10 Gew.%.

Die verwendete Sprühlösung besitzt im allgemeinen einen Polymeranteil von 5 bis 10 %.
Die Freisetzung des Wirkstoffes beginnt wie zuvor bei der Matrixtablette beschrieben bereits im Magen und kann durch Veränderung des Molekulargewichts über einen längeren Zeitraum variiert werden.
Durch Kombination mit anderen wasserlöslichen Polymeren, wie z.B. Polyethylenglykolen (z.B. Polyethylenglykol 6000), können die Freigaberaten variiert werden.

Gemäß einem weiteren Verfahren wird das feinteilige Polymermaterial mit dem Wirkstoff und gegebenenfalls mit Hilfsstoffen, wie z.B. Lactose, vermischt und anschließend extrudiert.
Die wirkstoffhaltigen Extrudate, z.B. Stäbchen, werden dann zu geeigneten galenischen Darreichungsformen weiterverarbeitet. Die nach dem sogenannten Extrusionsverfahren hergestellten Arzneimittel besitzen den Vorteil, daß bei ihrer Herstellung keine Lösunsmittel verwendet werden müssen.

Die erfindungsgemäßen Arzneimittelzubereitungen eignen sich außerdem zur Stabilisierung bestimmter Wirkstoffe in Arzneimitteln.

Einige pharmazeutische Wirkstoffe aus der Gruppe der Sympathikomimetika des Phenylethylamin-Typs neigen bei der Lagerung zu oxidativer Veränderung: insbesondere bei Feuchtezutritt können leicht bis stärker verfärbte Produkte entstehen. Erfahrungsgemäß sind derartige Veränderungen bei neutralem bis alkalischem pH-Wert gegenüber dem sauren Milieu begünstigt.
Säurezusatz zu derartigen Arzneimittlen ist nicht in jedem Falle das Mittel der Wahl. Polymere der Milchsäure hingegen, die unter Wasserzutritt hydrolytisch bis zum Monomer gespalten werden, können hier als latentes, bedarfsgesteuertes Säureresorvoir fungieren.

Ein Tablettengranulat, mit 7,5 % m-Proterenolsulfat (Warenzeichen: Alupent) verfärbt sich innerhalb von 48 Stunden, wenn es auf Granulatfeuchte verschlossen auf ca. 60°C gehalten wird. Ein gleichartiges Granulat, dem 5 % (Poly-L-Milchsäure), Molekulargewicht 2000, zugesetzt wurde, zeigt unter gleichartigen Bedingungen keine Verfärbung.

Die nachfolgenden Beispiele sollen die Erfindung näher erläutern.

Beispiel 1

Poly-D,L-Lactid (Polymerisationsgrad 4 bis 6), wird mit Methylenchlorid aufgenommen und mit 1 Gew.-% Clonidin-Base versetzt. Anschließend wird das Lösungsmittel abgezogen und der entstandene Film kalt vermahlen. Das erhaltene Mahlgut wird durch Siebung zu einer log-normalen Verteilung, z.B. mit den Parametern (dz = 80µm, $\sigma$ 0,23) (dz mittlerer Durchmesser der Teilchen, $\sigma$ Standardabweichung) zusammengestellt und die Freigabe des Clonidins mittels HPLC im USP Tester XVII vermessen. Bei der Bestimmung der Freigaberate des Clonidins aus diesem oligomerem Poly-Lactid wird in der Magensaftphase (pH 1,2; Verweildauer 1h) praktisch kein Arzneistoff freigesetzt, erst bei Übergang in den Darmsaft erfolgt dann die Freigabe des Arzneistoffes. In Tab. 1 sind die prozentualen Freigabedaten an Clonidin über einen Beobachtungszeitraum von 6 Stunden dargestellt.

Tabelle 1: Freigabedaten der Charge We T 66 im modifizierten USP Tester XVII

| | | | |
|---|---|---|---|
| 0,25 | h | 0,6 | % des freigegebenen Wirkstoffs |
| 1 | h | 0,8 | |
| 2 | h | 29,8 | |
| 4 | h | 55,6 | |
| 6 | h | 70,8 | |

Beispiel 2: Matrixtabletten

Theophyllin-Polymergranulat erhalten durch Feuchtgranulation mit organischem Lösungsmittel

Herstellung der Tabletten: Exzenterpresse EKO, Stempel:
12 mm Ø flach, Facette gewölbt.

Bestimmung der Freisetzungsrate:

USP 21 Tester, Paddle Modell, 100 bzw. 150 UpM, Puffer pH 1,2, pH 6,5

A

| Zusammensetzung | | Freigabe | |
|---|---|---|---|
| a) | Theophyllin 80% | 1h | 18,5% |
| | Poly(-D,L-lactid) 20% | 3h | 31,3% |
| | Mg.-stearat 0,3% | 5h | 40,1% |
| | Aerosil 0,3% | 7h | 47,1% |
| | Dichlormethan 40ml | | |

Bemerkungen:Tabletten sehen nach der Freigabe unverändert aus, sehr hart. Best. wurde bei 100 UpM durch geführt

| | | | |
|---|---|---|---|
| b) Theophyllingranulat | Theophyllin 80% | 1h | 21,1% |
| äußere Phase:Mg.-stearat 0,3% | Poly(D,L-lactid-co-glycolid) 50:50/20% | 3h | 36,0% |
| Aerosil 0,3% | | 5h | 46,6% |
| | Dichlormethan 20ml | 7h | 55,4% |

Bemerkungen:Nach der Freigabe sehen die Tabletten unverändert aus, sehr hart. Best. bei 100 UpM durch geführt

| | | | |
|---|---|---|---|
| c) Theophyllingranulat (0,8-1,0mm) | Theophyllin 80% | 1h | 24,3% |
| äußere Phase: | Poly(D,L-lactid-co-glycolid) 75:25/20% | 3h | 42,8% |
| Mg.-stearat 1,0% | | 5h | 54,9% |
| Aerosil 0,3% | Dichlormethan 50ml | 7h | 64,3% |

Best. wurde bei 150 UpM durchgeführt.

| | | | |
|---|---|---|---|
| Theophyllingranulat | Theophyllin 80% | 1h | 23,8% |
| (kleiner 0,3mm) | Poly(D,L-lactid) | 3h | 40,7% |
| | Dichlormethan 40 ml | 5h | 52,3% |
| | Aethanol 10 ml | 7h | 61,2% |

Bemerkungen:Die Tabletten waren nach der Freigabe noch stabil. Best. wurde bei 150 UpM durchgeführt.

| | | | Härte 1 | Härte 2 |
|---|---|---|---|---|
| Theophyllingranulat 100% | Theophyllin 80% | 1h | 25,3% | 21,1% |
| (kleiner 0,3mm) | Poly-L-lactid,M ca 2000 | 3h | 63,0% | 44,3% |
| | Dichlormethan 20ml | 5h | 86,2% | 61,5% |
| | | 7h | 95,6% | 74,8% |

Bemerkungen: Die Tabletten sind nach der Freigabe unverändert, Best. wurde bei 150 UpM durchgeführt.
Härte 1 = 140 kN, Härte 2 = 150 kN

Beispiel 3

POLYLACTIDE zum Überziehen von peroralen Darreichungsformen (allein sowie in Kombination mit anderen Polymeren: Polylethylenglykol 6000, Eudragit[R] NE 30 D. Die Angaben beziehen sich auf Trockensubstanz)

| | |
|---|---|
| Wirkstoff: | Theophyllin |
| Überzogene Form: | gerundete Pellets mit ca. 80 % Wirkstoff Größe: ca.1,0-1,6mm |
| Sprühung/Gerät: | WST 1 |
| Lösungsmittel: | Methylenchlorid |
| Ansatzgröße: | ca. 1,2 kg |
| Sprühlösung: | jeweils 5 % Polymer (falls nicht anders angegeben) |
| In vitro-Prüfmethode: | USP21 Tester (Paddle) 150 upm Puffer pH 1,2/pH 6,5 |

| Polymerzusammensetzung | Freigabe | | |
|---|---|---|---|
| | Überzug | 5 % | 10 % |
| 1.0.Poly-L-lactid, MG ca. 2000 100 % | 1h | | 43,8% |
| | 3h | | 91,7% |
| | 5h | | 97,5% |
| | 7h | | 98,5% |
| 1.1.Poly-L-lactid MG ca. 2000 75 % Eudragit NE30D 25% | 1h | 12,5% | 22,8% |
| | 3h | 33,5% | 44,2% |
| | 5h | 52,5% | 56,6% |
| | 7h | 68,4% | 65,7% |
| 1.2.Poly-L-lactid MG ca. 2000 50 % Eudragit NE30D 50% | 1h | 10,3% | 16,5% |
| | 3h | 30,1% | 37,9% |
| | 5h | 51,2% | 51,9% |
| | 7h | 66,3% | 61,8% |
| 1.3.Poly-L-lactid MG ca. 2000 75 % Polyethylenglykol 6000 25 % | 1h | | 100,0% |
| | 3h | | |
| | 5h | | |
| | 7h | | |

| | | Überzug 5% | 10% |
|---|---|---|---|
| 1.4. Poly-L-lactid 50%<br>Polyethylenglykol 6000 50 % | 1h<br>3h<br>5h<br>7h | | 100,0% |
| 2.0. Poly-L-Lactid inh. Visk.<br>0,9 dl/g 100 % | 1h<br>3h<br>5h<br>7h | | 55,6%<br>87,6%<br>94,5%<br>96,8% |
| 2.1. Poly-L-Lactid inh. Visk.<br>0,9 dl/g 94 %<br>Eudragit NE30D 6% | 1h<br>3h<br>5h<br>7h | 1,1%<br>2,9%<br>4,8%<br>6,5% | 0,9%<br>2,3%<br>1,9%<br>3,7% |
| 2.2. Poly-L-Lactid inh. Visk.<br>0,9 dl/g 88 %<br>Eudragit NE30D 12 % | 1h<br>3h<br>5h<br>7h | 3,7%<br>7,9%<br>11,9%<br>15,5% | 0,7%<br>1,9%<br>3,2%<br>4,6% |
| 2.3. Poly-L-Lactid inh. Visk.<br>0,9 dl/g 75 %<br>Eudragit NE30D 25%<br>Bemerkungen: Lacklösung 4 %ig | 1h<br>3h<br>5h<br>7h | 1,3%<br>3,0%<br>4,7%<br>6,3% | 1,3%<br>2,3%<br>3,2%<br>4,0% |
| 2.4. Poly-L-Lactid inh. Visk.<br>0,9 dl/g 50 %<br>Eudragit NE30D 50%<br>Bemerkungen: Lacklösungen 3%ig | 1h<br>3h<br>5h<br>7h | 1,5%<br>3,5%<br>5,5%<br>7,7% | 1,1%<br>2,4%<br>3,6%<br>5,0% |
| 2.5. Poly-L-Lactid inh. Visk.<br>0,9 dl/g 75 %<br>Polyethylenglykol 6000 25% | 1h<br>3h<br>5h<br>7h | 78,1%<br>94,3%<br>97,5%<br>98,5% | 20,9%<br>48,4%<br>64,9%<br>75,6% |

| | % Überzug | 5 % | 10 % |
|---|---|---|---|
| 2.6 Poly-L-Lactid inh. Visk. 0,9 dl/g | 1h | | 100,0% |
| | 3h | | |
| | 5h | | |
| | 7h | | |

Alle %-Angaben beziehen sich auf den tatsächlich gefundenen Gesamtgehalt. Beispiel 3 veranschaulicht die Freigaberaten bei 5 und 10 %igem Überzug. Die aufgeführten Polymeranteile ergeben in der Summe 100 %.

## Patentansprüche

1. Perorale wirkstoffhaltige Arzneimittelzubereitung mit kontrollierter, verzögerter Wirkstofffreigabe auf der Basis eines biologisch abbaubaren Trägermaterials und gegebenenfalls eines Überzugsmaterials ausgewählt aus der Gruppe Glykolid und/oder Lactid, L-Lactid, D-Lactid, D,L-Lactid, meso-Lactid, Poly-L-Lactid, Poly-D-Lactid, Poly-D,L-Lactid, Poly(L-Lactid-co-glycolid) und Poly(D,L-Lactid-co-glycolid) oder einer oder mehrerer Kombinationen daraus, hergestellt nach folgendem Verfahren
   - lösen bzw. suspendieren des Trägermaterials und 0.1 bis 20 % des Wirkstoffs in einem leichtflüchtigen Lösungsmittel, ausgießen der Lösung zu einem Film, diesen trocknen lassen und anschließend auf eine Korngröße von 50 bis 500 µm vermahlen und verpressen des so erhaltenen Pulvers - gegebenenfalls Zusatz von üblichen Hilfsstoffen und - zu Tabletten bzw. abfüllen in Kapseln.

2. Perorale wirkstoffhaltige Arzneimittelzubereitung mit kontrollierter, verzögerter Wirkstofffreigabe auf der Basis eines biologisch abbaubaren Trägermaterials und gegebenenfalls eines Überzugsmaterials ausgewählt aus der Gruppe Glykolid und/oder Lactid, L-Lactid, D-Lactid, D,L-Lactid, meso-Lactid, Poly-L-Lactid, Poly-D-Lactid, Poly-D,L-Lactid, Poly(L-Lactid-co-glycolid) und Poly(D,L-Lactid-co-glycolid) oder einer oder mehrerer Kombinationen daraus, hergestellt nach folgendem Verfahren
   - verpressen des Trägermaterials zusammen mit dem Wirkstoff, gegebenenfalls unter Zusatz von Hilfsstoffen und verpressen zu Tabletten nach Granulation.

3. Perorale wirkstoffhaltige Arzneimittelzubereitung mit kontrollierter, verzögerter Wirkstofffreigabe auf der Basis eines biologisch abbaubaren Trägermaterials und gegebenenfalls eines Überzugsmaterials ausgewählt aus der Gruppe Glykolid und/oder Lactid, L-Lactid, D-Lactid, D,L-Lactid, meso-Lactid, Poly-L-Lactid, Poly-D-Lactid, Poly-D,L-Lactid, Poly(L-Lactid-co-glycolid) und Poly(D,L-Lactid-co-glycolid) oder einer oder mehrerer Kombinationen daraus, hergestellt nach folgendem Verfahren
   - herstellen einer Lösung bestehend aus Trägermaterial und einem organischen Lösungsmittel und aufbringen dieser Lösung auf ein Granulat oder auf Pellets, so daß der Anteil des Überzuges zwischen 3 und 30 Gew.-% beträgt.

4. Perorale wirkstoffhaltige Arzneimittelzubereitung nach Anspruch 3, dadurch gekennzeichnet, daß der Anteil des Überzuges zwischen 5 und 15 Gew.-% beträgt.

## Claims

1. Oral pharmaceutical preparation containing active substance, with a controlled, delayed release of active substance, based on a biodegradable carrier material and optionally a coating material, selected from the group comprising glycolide and/or lactide, L-lactide, D-lactide, DL-lactide, meso-lactide, poly-L-lactide, poly-D-lactide, poly-DL-lactide, poly(L-lactide-co-glycolide) and poly(DL-lactide-co-glycolide) or one or more combinations thereof, prepared by the following method:
   the carrier material together with 0.1 to 20% of the active substance is dissolved or suspended in a volatile solvent, poured out to form a film and left to dry, this is then ground to a particle size of 50 to

500 $\mu$m and the resulting powder, optionally with the addition of convention excipients, is packed into capsules or compressed to form tablets.

**2.** Oral pharmaceutical preparation containing active substance, with a controlled, delayed release of active substance, based on a biodegradable carrier material and optionally a coating material, selected from the group comprising glycolide and/or lactide, L-lactide, D-lactide, DL-lactide, meso-lactide, poly-L-lactide, poly-D-lactide, poly-DL-lactide, poly(L-lactide-co-glycolide) and poly(DL-lactide-co-glycolide) or one or more combinations thereof, prepared by the following method:
the carrier material is compressed together with the active substance, optionally with the addition of excipients, to form tablets, after being granulated.

**3.** Oral pharmaceutical preparation containing active substance, with a controlled, delayed release of active substance, based on a biodegradable carrier material and optionally a coating material, selected from the group comprising glycolide and/or lactide, L-lactide, D-lactide, DL-lactide, meso-lactide, poly-L-lactide, poly-D-lactide, poly-DL-lactide, poly(L-lactide-co-glycolide) and poly(DL-lactide-co-glycolide) or one or more combinations thereof, prepared by the following method:
a solution is prepared from the carrier material and an organic solvent and is applied to granules or pellets so that the proportion of coating is between 3 and 30% by weight.

**4.** Oral pharmaceutical preparation containing an active substance according to claim 3, characterised in that the amount of coating is between 5 and 15% by weight.

**Revendications**

**1.** Préparation médicamenteuse pour administration par voie buccale contenant un principe actif à libération contrôlée, retardée du principe actif, à base d'un matériau support biologiquement dégradable et éventuellement d'un matériau de revêtement choisis dans le groupe formé par un glycolide et/ou un lactide, L-lactide, D-lactide, D,L-lactide, méso-lactide, poly-L-lactide, poly-D-lactide, poly-D,L-lactide, un copolymère L-lactide-glycolide et un copolymère D,L-lactide-glycolide ou par une ou plusieurs combinaisons de ceux-ci, préparée selon le procédé suivant:
- dissolution ou mise en suspension du matériau support et de 0,1 à 20% de principe actif dans un solvant volatil, coulée de la solution en un film, séchage de celui-ci puis broyage à une taille de grains de 50 à 500 $\mu$m et compactage de la poudre ainsi obtenue, éventuellement addition d'adjuvants habituels, en comprimés ou introduction dans des capsules.

**2.** Préparation médicamenteuse pour administration par voie buccale contenant un principe actif à libération contrôlée, retardée du principe actif à base d'un matériau support biologiquement dégradable et éventuellement d'un matériau de revêtement choisis dans le groupe formé par un glycolide et/ou un lactide, L-lactide, D-lactide, D,L-lactide, méso-lactide, poly-L-lactide, poly-D-lactide, poly-D,L-lactide, un copolymère L-lactide-glycolide et un copolymère D,L-lactide-glycolide ou par une ou plusieurs combinaisons de ceux-ci, préparée selon le procédé suivant:
- compactage du matériau support et du principe actif, éventuellement avec addition d'adjuvants et compactage en comprimés après granulation.

**3.** Préparation médicamenteuse pour administration par voie buccale contenant un principe actif à libération contrôlée, retardée du principe actif à base d'un matériau support biologiquement dégradable et éventuellement d'un matériau de revêtement choisis dans le groupe formé par un glycolide et/ou un lactide, L-lactide, D-lactide, D,L-lactide, méso-lactide, poly-L-lactide, poly-D-lactide, poly-D,L-lactide, un copolymère L-lactide-glycolide et un copolymère D,L-lactide-glycolide ou par une ou plusieurs combinaisons de ceux-ci, préparée selon le procédé suivant:
- préparation d'une solution consistant en le matériau support et en un solvant organique et application de cette solution sur des granulés ou sur des pastilles de sorte que la proportion de revêtement soit comprise entre 3 et 30% en poids.

**4.** Préparation médicamenteuse pour administration par voie buccale contenant un principe actif selon la revendication 3, caractérisée en ce que la proportion de revêtement est comprise entre 5 et 15% en poids.